# EUROPEAN PATENT SPECIFICATION

(11) **EP 3 285 672 B1**
(45) Date of publication and mention of the grant of the patent: **03.04.2019**
(21) Application number: 15802306.9
(22) Date of filing: 18.11.2015
(51) Int. Cl.: A61B 17/88, A61B 17/34

(54) **SURGICAL ASSEMBLY FOR REPAIRING DEPRESSION FRACTURES**
CHIRURGISCHE ANORDNUNG ZUR REPARATUR VON DEPRESSIONSFRAKTUREN
ASSEMBLAGE CHIRURGICAL POUR RÉPARER DES FRACTURES À DÉPRESSION

(30) Priority: 21.04.2015 US 201514691963
(43) Date of publication of application: 28.02.2018
(73) Proprietor: Arthrex, Inc., Naples, FL 34108-1945 (US)
(72) Inventor: ELSER, Christoph, 85665 Moosach (DE); SCHIFFER, Niclas W., 80335 Munchen (DE); ROLLER, Brandon, Winston-Salem, NC 27106 (US); CHAUDOT, Audrey, 81675 Munchen (DE); PARRATTE, Sebastien, 13008 Marseille (FR); DINES, Joshua, Southampton, New York 11968 (US)
(74) Representative: Rankin, Douglas
(86) International application number: PCT/US2015/061212
(87) International publication number: WO 2016/171762

(56) References cited:
- US-A1- 2007 198 024
- US-A1- 2011 015 675
- US-A1- 2012 059 380
- US-A1- 2012 316 513

## Description

### BACKGROUND

This disclosure relates to a surgical assembly and method for reducing depression fractures. US2011/0015675 describes a system for implanting an anchor into bone, the system comprising a curved cannulated guide for percautaneous insertion, having a proximal end and a distal end; a flexible drill insertable through the curved guide from the proximal end to the distal end, the flexible drill having a shaft having a flexible portion; and a flexible inserter for inserting a suture anchor into a bore at the anatomical site formed by the flexible drill, the flexible inserter having a shaft having a flexible portion, wherein the flexible portions of both the flexible drill and flexible inserter include a series of discrete, interlocking segments.

Prolonged periods of high force impact and stress during sporting activities can result in depression fractures in the articulating surfaces of joints. Collapsed bone fragments associated with the depression fracture can depress into softer cancellous bone located beneath relatively hard cortical bone. The collapsed bone fragments must be returned to their original anatomical position to stabilize the joint and minimize the risk of post traumatic osteoarthritis.

### SUMMARY

The present invention is defined in claim 1. A surgical assembly according to an exemplary aspect of the present disclosure includes, among other things, a cannula and an obturator receivable through the cannula and configured to manipulate a depressed bone fragment associated with a depression fracture.

The obturator includes a blunt tip that extends beyond a distal end of the cannula.

In a further non-limiting embodiment of either of the foregoing surgical assemblies, the blunt tip is rounded.

The blunt tip and the distal end are curved and extend along an axis that is transverse to a longitudinal axis of the surgical assembly.

In a further non-limiting embodiment of any of the foregoing surgical assemblies, the cannula includes a tube that extends between a fitting and a distal end.

In a further non-limiting embodiment of any of the foregoing surgical assemblies, the tube includes a passage that extends between the fitting and the distal end.

In a further non-limiting embodiment of any of the foregoing surgical assemblies, a plurality of openings are formed through the tube at the distal end.

In a further non-limiting embodiment of any of the foregoing surgical assemblies, the obturator includes a shaft that extends between a handle and a blunt tip.

In a further non-limiting embodiment of any of the foregoing surgical assemblies, the handle includes a fitting and a grip that extends from the fitting.

The obturator is removable from the cannula.

The assembly includes a delivery device for injecting a repair material through the cannula after removing the obturator.

In a further non-limiting embodiment of any of the foregoing surgical assemblies, the cannula includes a first fitting and the obturator includes a second fitting configured to engage the first fitting to connect the obturator to the cannula.

A surgical method for using the present invention includes, among other things, accessing a depressed bone fragment of a depression fracture with a surgical assembly. The surgical assembly includes a cannula and an obturator receivable through the cannula. The method further includes positioning the depressed bone fragment toward its original anatomical position using a blunt tip of the obturator.

In a further example of the foregoing surgical method, the accessing step includes locating the blunt tip of the obturator beneath the depressed bone fragment.

In a further example of either of the foregoing surgical methods, the locating step includes positioning the blunt tip such that it is generally perpendicular to the depressed bone fragment.

In a further example of any of the foregoing surgical methods, the method includes, prior to the accessing step, forming a pilot hole through cortical bone that is located inferior to the depression fracture.

In a further example of any of the foregoing surgical methods, the method includes, subsequent to the positioning step, removing the obturator from the cannula.

In a further non-limiting embodiment of any of the foregoing surgical methods, the method includes injecting a repair material through the cannula after the removing step.

In a further example of any of the foregoing surgical methods, the blunt tip extends past a distal end of the cannula.

In a further example of any of the foregoing surgical methods, the positioning step includes raising the depressed bone fragment toward the original anatomical position.

The embodiments, examples and alternatives of the preceding paragraphs, or the following description and drawings, including any of their various aspects or respective individual features, may be taken independently or in any combination. Features described in connection with one embodiment are applicable to all embodiments, unless such features are incompatible.

Accordingly, a surgical assembly according to an exemplary aspect of the present disclosure includes a cannula and an obturator receivable through the cannula and configured to manipulate a depressed bone fragment associated with a depression fracture.

In the surgical assembly, the obturator includes a blunt tip that extends beyond a distal end of the cannula. In the surgical assembly, the cannula may include a tube that extends between a fitting and a distal end. The tube includes a passage that extends between the fitting and the distal end. In the surgical assembly, the obturator may include a shaft that extends between a handle and a blunt tip. In the surgical assembly, the cannula may include a tube that extends between a fitting and a distal end and the obturator may include a shaft that extends between a handle and a blunt tip. In the surgical assembly, the obturator includes a blunt tip that extends beyond a distal end of the cannula, the cannula may include a tube that extends between a fitting and a distal end and the obturator may include a shaft that extends between a handle and a blunt tip. In the surgical assembly, the obturator is removable from the cannula. In the surgical assembly, the cannula may include a first fitting and the obturator may include a second fitting configured to engage the first fitting to connect the obturator to the cannula. In the surgical assembly, the obturator includes a blunt tip that extends beyond a distal end of the cannula and the obturator is removable from the cannula and/or the cannula may include a first fitting and the obturator may include a second fitting configured to engage the first fitting to connect the obturator to the cannula. The surgical assembly includes a delivery device for injecting a repair material through the cannula after removing the obturator. For example, in the surgical assembly, the obturator includes a blunt tip that extends beyond a distal end of the cannula, the cannula includes a tube that extends between a fitting and a distal end, the tube including a passage that extends between the fitting and the distal end, the obturator includes a shaft that extends between a handle and a blunt tip, and the obturator is removable from the cannula, for example where the cannula includes a first fitting and the obturator includes a second fitting configured to engage the first fitting to connect the obturator to the cannula. Such an assembly may also include, for example, a delivery device for injecting a repair material through the cannula after removing the obturator.

In a surgical assembly also having one or more of these further features, the blunt tip may be rounded, the blunt tip and the distal end of the cannula are curved and extend along an axis that is transverse to a longitudinal axis of the surgical assembly, a plurality of openings may be formed through the tube at the distal end, and the handle of the obturator may include a fitting and a grip that extends from the fitting. For example, in the surgical assembly, the obturator includes a rounded blunt tip that extends beyond a distal end of the cannula, the blunt tip and the distal end of the cannula are curved and extend along an axis that is transverse to a longitudinal axis of the surgical assembly, the cannula includes a tube that extends between a fitting and a distal end, the tube including a passage that extends between the fitting and the distal end, where a plurality of openings are formed through the tube at the distal end, the obturator includes a shaft that extends between a handle and a blunt tip, where the handle of the obturator includes a fitting and a grip that extends from the fitting, and the obturator is removable from the cannula, for example where the cannula includes a first fitting and the obturator includes a second fitting configured to engage the first fitting to connect the obturator to the cannula. Such an assembly includes a delivery device for injecting a repair material through the cannula after removing the obturator.

A surgical method includes accessing a depressed bone fragment of a depression fracture with a surgical assembly, where the surgical assembly includes a cannula and an obturator receivable through the cannula, and the method further includes positioning the depressed bone fragment toward its original anatomical position using a blunt tip of the obturator.

In the surgical method, the accessing step may include locating the blunt tip of the obturator beneath the depressed bone fragment. The surgical method may include, prior to the accessing step, forming a pilot hole through cortical bone that is located inferior to the depression fracture. The surgical method may include, subsequent to the positioning step, removing the obturator from the cannula. The surgical method may include locating the blunt tip of the obturator beneath the depressed bone fragment and may also include, prior to the accessing step, forming a pilot hole through cortical bone that is located inferior to the depression fracture and/or, subsequent to the positioning step, removing the obturator from the cannula. The surgical method may also include injecting a repair material through the cannula after the removing step.

In a surgical method also having any one or more of these further features, the locating step may include positioning the blunt tip such that it is generally perpendicular to the depressed bone fragment and the positioning step may include raising the depressed bone fragment toward the original anatomical position, and the blunt tip of the obturator may extend past a distal end of the cannula. For example, the surgical method includes locating the blunt tip of the obturator beneath the depressed bone fragment, where the blunt tip extends past a distal end of the cannula, where the locating step includes positioning the blunt tip such that it is generally perpendicular to the depressed bone fragment and the positioning step includes raising the depressed bone fragment toward the original anatomical position, and also includes, prior to the accessing step, forming a pilot hole through cortical bone that is located inferior to the depression fracture and/or, subsequent to the positioning step, removing the obturator from the cannula and optionally further injecting a repair material through the cannula after removing the obturator.

Aspects, features and embodiments of the present disclosure are described further below.

For example, an exemplary surgical assembly of the present disclosure includes a cannula and an obturator removably received within the cannula. A passage may extend entirely through the cannula. In other words, the cannula is cannulated. The obturator is insertable through the passage. The obturator may be removably attachable to the cannula. The obturator includes a blunt tip that extends past a distal end of the cannula. The cannula may include a tube that extends along a longitudinal axis of the surgical assembly between a fitting, disposed proximally, and the distal end. The fitting of the cannula may include a grip. The obturator may include a shaft that extends along the longitudinal axis between a handle and the blunt tip. The handle of the obturator may include a fitting and a grip.

The surgical assembly is for reducing a depression fracture, such as of a musculoskeletal joint. The obturator which is removably receivable within the cannula is configured to position a depressed bone fragment, such that the depressed bone fragment may be accessed and elevated to its original anatomical position using the surgical assembly. Thus, the obturator is insertable through the passage to access depressed bone fragments associated with a depression fracture. The blunt tip is configured to position depressed bone fragment(s) associated with a depression fracture. The fitting of the cannula is configured for connection to the obturator. The grip of the cannula fitting is configured for gripping the cannula. The fitting of the obturator is adapted to connect to the fitting of the cannula and the grip of the obturator is configured for gripping the surgical assembly.

The cannula and obturator may be connected together using a luer connection. Other connections, however, are also contemplated within the scope of this disclosure. The blunt tip may be configured as a non-traumatic tip designed to avoid damaging the depressed bone fragments. For example, the blunt tip is rounded. However, other non-traumatic shapes are also contemplated within the scope of this disclosure. Both the distal end of the cannula and the blunt tip of the obturator are curved. For example, the distal end and the blunt tip are both curved such that they extend at a transverse angle relative to a longitudinal axis of the surgical assembly. The actual angle of the curvature may vary depending on the joint being repaired, among other factors. The distal end of the cannula and the blunt tip of the obturator may be pre-bent or can be bent in the operating room just prior to performing a depression fracture reduction surgery. At the distal end of the cannula, there may be a plurality of openings, or fenestrations, formed through the tube. The plurality of openings are in fluid communication with the passage that extends through the tube. The fitting of the cannula configured for connection to the obturator, is, for example, a male luer lock fitting that may be rotatably received within a female luer lock fitting of the obturator. Other fitting configurations are also contemplated, including an example in which the fitting of the cannula provides the female connection and the obturator provides the male connection. The handle of the obturator may include any size, shape and/or configuration. In other words, the handle is not limited to specific exemplary configurations.

The cannula may be made of a metallic material, such as stainless steel. The cannula may be made of a non-metallic material, such as a plastic. The tube and the fitting of the cannula may be made of either similar or dissimilar materials.

The obturator may be made of a metallic material, such as stainless steel. Other metallic materials may also be suitable. The shaft and the fitting of the obturator may be made of similar or dissimilar materials.

For example, an exemplary surgical method for reducing a depression fracture of a joint of the present disclosure includes accessing the depressed bone fragment and elevating it to its original anatomical position using a surgical assembly of the present disclosure.

For example, the joint is a knee joint that includes a femur and a tibia and the depression fracture is a depression fracture of the tibial plateau. However, the depression fracture could alternatively be a fracture associated with a Hill Sachs lesion, the calcaneus, the distal radius, or any other bone or joint. In this disclosure, the phrase "depression fracture" indicates a fracture in which one or more bone fragments depress into the softer cancellous bone located beneath relatively hard cortical bone. The depressed bone fragment(s) must be returned to their original anatomical position to stabilize the joint. The surgical method of the present disclosure may be performed as either an arthroscopic method or an open method.

The method may include forming a pilot hole through the cortical bone. The method may include guidance of the surgical assembly through the pilot hole. The method may include manipulating the depressed bone fragment(s) back to an original anatomical position. The method may include disconnecting the obturator from the cannula and then removing the obturator from the joint after reducing the depressed bone fragments. The method may include injecting into the joint a repair material to backfill the hollow area immediately beneath the bone fragment(s) of the depression fracture. The method may include removing the cannula from the joint to complete the depression fracture reduction procedure.

For example, a pilot hole may be formed through the cortical bone. The pilot hole provides an opening through which the depressed bone fragment(s) associated with the depression fracture may be accessed using the surgical assembly. In one example, the pilot hole is formed at approximately a 45° angle at a location of the cortical bone that is inferior to (i.e., below) the depression fracture. In another example, the pilot hole is formed on the same side of the joint as the depression fracture. For example, the pilot hole is formed on the medial side of the tibia if the depression fracture is located medially and is formed on the lateral side of the tibia if the depression fracture is located laterally. The location of the pilot hole may be chosen with the aid of fluoroscopic imaging techniques that provide a real-time understanding of the positioning of the depressed bone fragments and the various other structures that make-up the joint.

For example, for guidance of a surgical assembly of the present disclosure through the pilot hole for accessing the depressed bone fragment(s), the tube of the cannula may include an outer diameter that is smaller than the pilot hole to ease insertion of the surgical assembly into the joint. The handle of the obturator and/or the grip of the cannula may be used to guide the surgical assembly to a desired positioning beneath the depressed bone fragment(s). The handle and/or the grip also provide sufficient leverage for inserting and guiding the surgical assembly through the cancellous bone to access the depression fracture.

After a desired positioning is achieved, the surgical assembly may be used to manipulate the depressed bone fragment(s) back to an original anatomical position. For example, the blunt tip of the obturator may be used to reduce the depressed bone fragment(s) as close as is possible back to an original anatomical position. The handle of the obturator and/or the grip of the cannula provide sufficient leverage for lifting the depressed bone fragments of the depression fracture. In one example, by virtue of the curved nature of the distal end of the cannula and the blunt tip of the obturator, the blunt tip may approach the depression fracture at a perpendicular angle relative to the depressed bone fragments. Approaching the bone fragments at such an angle simplifies manipulation of the depressed bone fragments for improved approximation back toward the original anatomical position.

The obturator may be disconnected from the cannula and then removed from the joint after reducing the depressed bone fragments. The cannula, however, may be left inside the joint for performing additional surgical steps.

For example, a repair material may be injected into the joint to backfill the hollow area immediately beneath the bone fragments of the depression fracture, which have been lifted back toward their original anatomical position. The repair material may be delivered using a delivery device that can be inserted into and through the cannula. The delivery device may include a syringe and tube, in one example. During injection, the repair material may flow through the passage of the cannula and then out of the openings at the distal end as well as the open distal end. The repair material may include resorbable or non-resorbable bone cement, a bone graft, a bone plug allograft or an autologous material. These, of course, are intended as non-limiting examples of suitable materials. The cannula is removed from the joint to complete the depression fracture reduction procedure.

As is apparent to those skilled in the art from the foregoing disclosure, while different non-limiting embodiments are illustrated as having specific components, the embodiments of this disclosure are not limited to those particular combinations. It is possible to use some of the components or features from any of the non-limiting embodiments in combination with features or components from any of the other non-limiting embodiments.

The various features and advantages of this disclosure will become apparent to those skilled in the art from the following detailed description. The drawings that accompany the detailed description can be briefly described as follows.

### BRIEF DESCRIPTION OF THE DRAWINGS

Figure 1 illustrates a surgical assembly according to a first embodiment of this disclosure.
Figure 2 illustrates a blown up view of region R1 of Figure 1.
Figure 3 illustrates a cannula of the surgical assembly of Figure 1.
Figure 4 illustrates an obturator of the surgical assembly of Figure 1.
Figure 5 illustrates portions of a surgical assembly according to another embodiment of this disclosure.
Figures 6, 7, 8, 9 and 10 schematically illustrate a method of reducing a depression fracture.

### DETAILED DESCRIPTION

This disclosure details surgical assemblies and associated methods for reducing a depression fracture of a musculoskeletal joint. The surgical assembly includes a cannula and an obturator receivable through the cannula and configured to position a depressed bone fragment. The depressed bone fragment may be accessed and elevated to its original anatomical position using the surgical assembly. These and other features are described in greater detail on the following paragraphs of this detailed description.

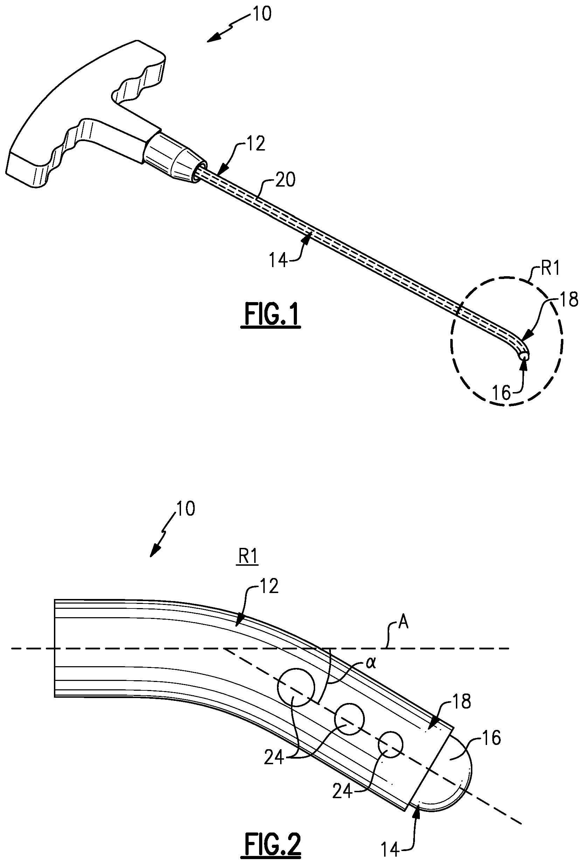

Figures 1 and 2 illustrate a surgical assembly 10 for reducing a depression fracture. The surgical assembly 10 includes a cannula 12 and an obturator 14 removably received within the cannula 12. A passage 20 may extend entirely through the cannula 12. In other words, the cannula 12 is cannulated. The obturator 14 is insertable through the passage 20 to access depressed bone fragments associated with a depression fracture. In one non-limiting embodiment, the obturator 14 is removably attachable to the cannula 12. For example, the cannula 12 and obturator 14 may be connected together using a luer connection. Other connections; however, are also contemplated within the scope of this disclosure.

The obturator 14 includes a blunt tip 16 that extends past a distal end 18 of the cannula 12. The blunt tip 16 is configured to position depressed bone fragment(s) associated with a depression fracture. In an embodiment, the blunt tip 16 is configured as a non-traumatic tip designed to avoid damaging the depressed bone fragments.

Referring primarily to Figure 2, which is a blown up view of region R1 of Figure 1, both the distal end 18 of the cannula 12 and the blunt tip 16 of the obturator 14 are curved. In an embodiment, the distal end 18 and the blunt tip 16 are both curved such that they extend at a transverse angle α relative to a longitudinal axis A of the surgical assembly 10. The actual angle of the curvature may vary depending on the joint being repaired, among other factors. The distal end 18 of the cannula 12 and the blunt tip 16 of the obturator 14 may be pre-bent or can be bent in the operating room just prior to performing a depression fracture reduction surgery.

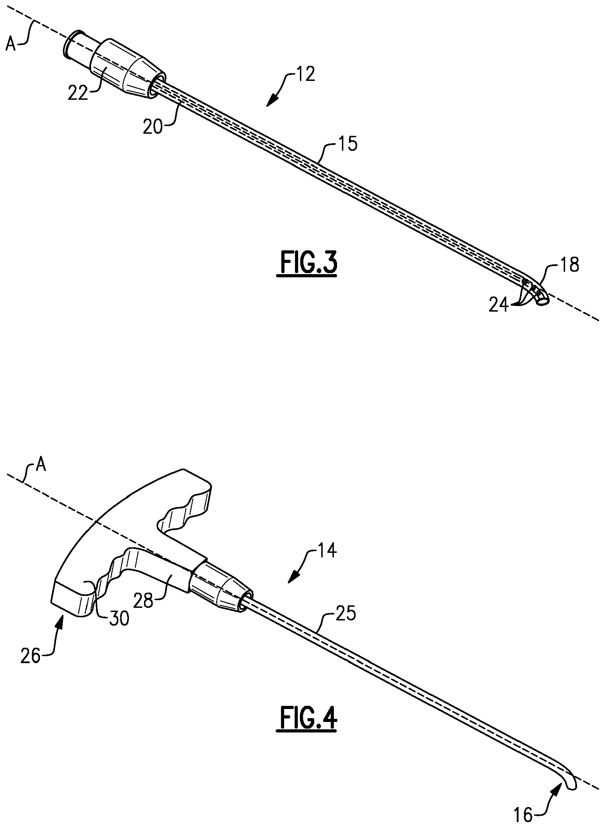

Figure 3, with continued reference to Figures 1 and 2, further illustrates the cannula 12 of the surgical assembly 10. The cannula 12 includes a tube 15 that extends along the longitudinal axis A between a fitting 22, disposed proximally, and the distal end 18. The distal end 18 of the cannula 12 may include a plurality of openings 24, or fenestrations, formed through the tube 15. The plurality of openings 24 are in fluid communication with the passage 20 that extends through the tube 15.

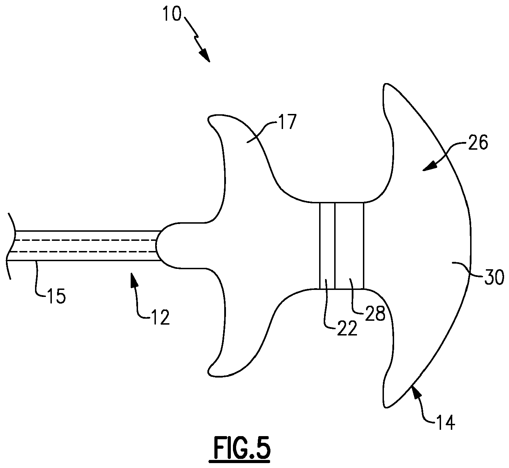

The fitting 22 is configured for connection to the obturator 14. In one non-limiting embodiment, the fitting 22 is a male luer lock fitting that may be rotatably received within a female luer lock fitting of the obturator 14. Other fitting configurations are also contemplated, including an embodiment in which the fitting 22 of the cannula 12 provides the female connection and the obturator 14 provides the male connection. In yet another embodiment, the fitting 22 includes a grip 17 for gripping the cannula 12 (see, for example, the embodiment of Figure 5).

In one non-limiting embodiment, the cannula 12 is made of a metallic material, such as stainless steel. In another embodiment, the cannula 12 may be made of a non-metallic material, such as a plastic. The tube 15 and the fitting 22 of the cannula 12 may be made of either similar or dissimilar materials.

Figure 4, with continued reference to Figures 1 and 2, further illustrates the obturator 14 of the surgical assembly 10. The obturator 14 includes a shaft 25 that extends along the longitudinal axis A between a handle 26 and the blunt tip 16. In one non-limiting embodiment, the blunt tip 16 is rounded. However, other non-traumatic shapes are also contemplated within the scope of this disclosure.

The handle 26 may include any size, shape and/or configuration. In other words, the handle 26 is not limited to the specific configurations shown in Figure 4 or Figure 5, which illustrate two non-limiting handle configurations. The handle 26 may include a fitting 28 and a grip 30. The fitting 28 is adapted to connect to the fitting 22 of the cannula 12 (see Figures 1 and 5) and the grip 30 is configured for gripping the surgical assembly 10.

In one non-limiting embodiment, the obturator 14 is made of a metallic material, such as stainless steel. Other metallic materials may also be suitable. The shaft 25 and the fitting 28 of the obturator 14 may be made of similar or dissimilar materials.

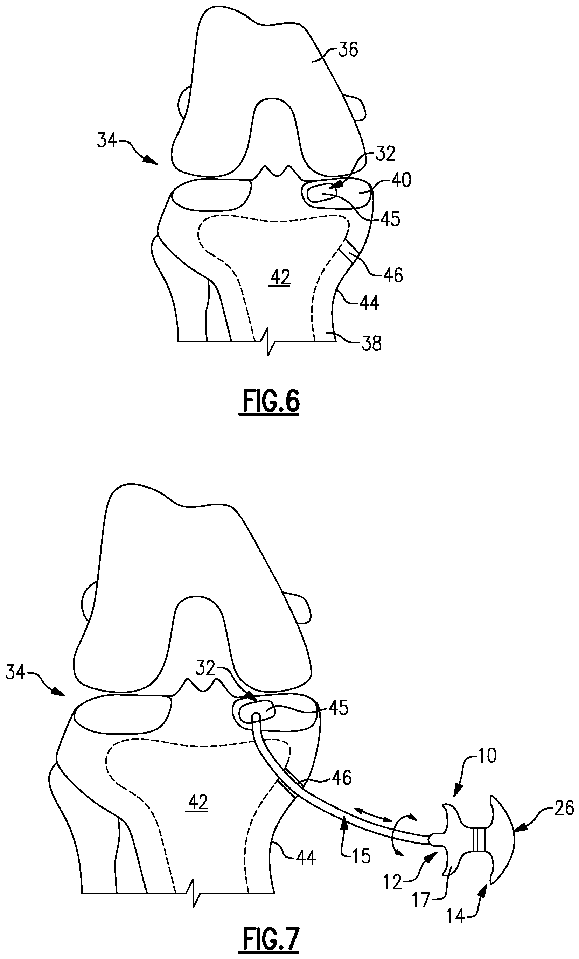

Figures 6-10, with continued reference to Figures 1-5, schematically illustrate a method for reducing a depression fracture 32 of a joint 34. In one non-limiting example, the joint 34 is a knee joint that includes a femur 36 and a tibia 38 and the depression fracture 32 is a depression fracture of the tibial plateau 40. However, the depression fracture 32 could alternatively be a fracture associated with a Hill Sachs lesion, the calcaneus, the distal radius, or any other bone or joint. In this disclosure, the phrase "depression fracture" indicates a fracture in which one or more bone fragments 45 depress into the softer cancellous bone 42 located beneath relatively hard cortical bone 44. The depressed bone fragment(s) 45 must be returned to their original anatomical position to stabilize the joint 34. The method shown in Figures 6-10 may be performed as either an arthroscopic method or an open method.

Referring first to Figure 6, a pilot hole 46 is formed through the cortical bone 44. The pilot hole 46 provides an opening through which the depressed bone fragment(s) 45 associated with the depression fracture 32 may be accessed using the surgical assembly 10. In one non-limiting example, the pilot hole 46 is formed at approximately a 45° angle at a location of the cortical bone 44 that is inferior to (i.e., below) the depression fracture 32. In another example, the pilot hole 46 is formed on the same side of the joint 34 as the depression fracture 32. For example, the pilot hole 46 is formed on the medial side of the tibia 38 if the depression fracture 32 is located medially and is formed on the lateral side of the tibia 38 if the depression fracture 32 is located laterally. The location of the pilot hole 46 may be chosen with the aid of fluoroscopic imaging techniques that provide a real-time understanding of the positioning of the depressed bone fragments 45 and the various other structures that make-up the joint 34.

Figure 7 schematically illustrates guidance of the surgical assembly 10 through the pilot hole 46 for accessing the depressed bone fragment(s) 45. In an embodiment, the tube 15 of the cannula 12 includes an outer diameter that is smaller than the pilot hole 46 to ease insertion of the surgical assembly 10 into the joint 34. The handle 26 of the obturator 14 and/or the grip 17 of the cannula 12 may be used to guide the surgical assembly 10 to a desired positioning beneath the depressed bone fragment(s) 45. The handle 26 and/or the grip 17 also provide sufficient leverage for inserting and guiding the surgical assembly 10 through the cancellous bone 42 to access the depression fracture 32.

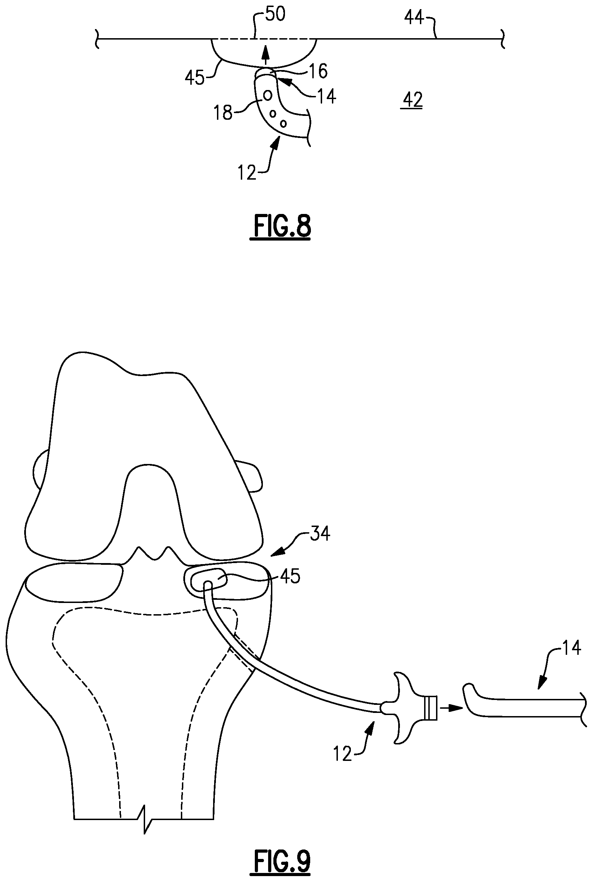

After a desired positioning is achieved, the surgical assembly 10 may be used to manipulate the depressed bone fragment(s) 45 back to an original anatomical positon. For example, as best illustrated in Figure 8, the blunt tip 16 of the obturator 14 may be used to reduce the depressed bone fragment(s) 45 as close as is possible back to an original anatomical position 50 (shown schematically in dashed lines in Figure 8). The handle 26 of the obturator 14 and/or the grip 17 of the cannula 12 provide sufficient leverage for lifting the depressed bone fragments 45 of the depression fracture 32. In one non-limiting example, by virtue of the curved nature of the distal end 18 of the cannula 12 and the blunt tip 16 of the obturator 14, the blunt tip 16 may approach the depression fracture 32 at a perpendicular angle relative to the depressed bone fragments 45. Approaching the bone fragments 45 at such an angle simplifies manipulation of the depressed bone fragments 45 for improved approximation back toward the original anatomical position 50.

As shown in Figure 9, the obturator 14 may be disconnected from the cannula 12 and then removed from the joint 34 after reducing the depressed bone fragments 45. The cannula 12, however, may be left inside the joint 34 for preforming additional surgical steps.

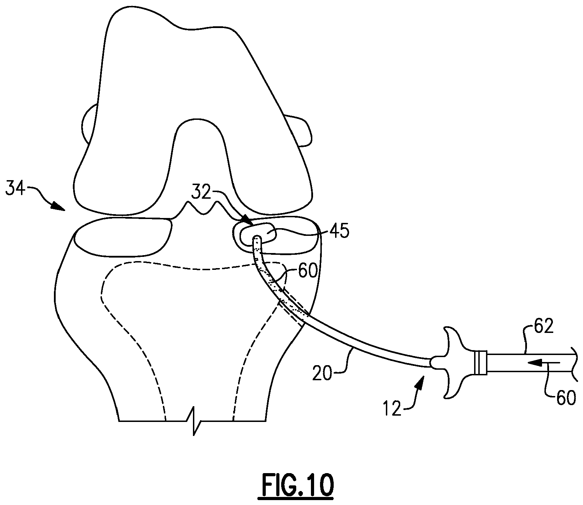

For example, as shown in Figure 10, a repair material 60 may be injected into the joint 34 to backfill the hollow area immediately beneath the bone fragments 45 of the depression fracture 32, which have not been lifted back toward their original anatomical position. The repair material 60 is delivered using a delivery device 62 that can be inserted into and through the cannula 12. The delivery device 62 may include a syringe and tube, in one non-limiting embodiment. During injection, the repair material 60 may flow through the passage 20 of the cannula 12 and then out of the openings 24 as well as the open distal end 18 (see Figure 3).

The repair material 60 may include resorbable or non-resorbable bone cement, a bone graft, a bone plug allograft or an autologous material. These, of course, are intended as non-limiting examples of suitable materials. The cannula 12 is removed from the joint 34 to complete the depression fracture reduction procedure.

Although the different non-limiting embodiments are illustrated as having specific components, the embodiments of this disclosure are not limited to those particular combinations. It is possible to use some of the components or features from any of the non-limiting embodiments in combination with features or components from any of the other non-limiting embodiments.

It should be understood that like reference numerals identify corresponding or similar elements throughout the several drawings. It should also be understood that although a particular component arrangement is disclosed and illustrated in these exemplary embodiments, other arrangements could also benefit from the teachings of this disclosure.

The foregoing description shall be interpreted as illustrative and not in any limiting sense. A worker of ordinary skill in the art would understand that certain modifications could come within the scope of this disclosure. For these reasons, the following claims should be studied to determine the true scope of the invention.

## Claims

1. A surgical assembly (10), comprising:
a cannula (12); and
an obturator (14) receivable through said cannula (12) and configured to manipulate a depressed bone fragment associated with a depression fracture,
wherein said obturator (14) includes a blunt tip (16) that extends beyond a distal end (18) of said cannula (12),
wherein said blunt tip (16) and said distal end (18) are curved and extend along an axis that is transverse to a longitudinal axis of said surgical assembly (10); wherein said obturator (14) is removable from said cannula (12), and wherein the surgical assembly includes:
a delivery device (62) configured for injecting a repair material (60) through said cannula (12), wherein said cannula (12) is adapted to receive said delivery device (62) when said obturator (14) is removed.

2. The surgical assembly as recited in claim 1, wherein said blunt tip (16) is rounded.

3. The surgical assembly as recited in claim 1, wherein said cannula (12) includes a tube (15) that extends between a fitting (22) and a distal end (18).

4. The surgical assembly as recited in claim 3, wherein said tube (15) includes a passage (20) that extends between said fitting (22) and said distal end (18).

5. The surgical assembly as recited in claim 3, comprising a plurality of openings (24) formed through said tube (15) at said distal end (18).

6. The surgical assembly as recited in claim 1, wherein said obturator (14) includes a shaft (25) that extends between a handle (26) and a blunt tip (16).

7. The surgical assembly as recited in claim 6, wherein said handle (26) includes a fitting (28) and a grip (30) that extends from said fitting.

8. The surgical assembly as recited in claim 1, wherein said cannula (12) includes a first fitting (22) and said obturator (14) includes a second fitting (28) configured to engage said first fitting (22) to connect said obturator (14) to said cannula (12).

9. The surgical assembly as recited in claim 1, wherein said delivery device (62) includes a syringe and a tube.

10. The surgical assembly as recited in claim 1, wherein said repair material (60) includes a bone cement, a bone graft, a bone plug allograft, or an autologous material.

11. The surgical assembly as recited in claim 5, wherein said plurality of openings (24) are in fluid communication with a passage (20) of said tube (15) such that said repair material (60) flows from said delivery device (62), through said passage (20), and then through said plurality of openings (24).

## Patentansprüche

1. Chirurgische Baugruppe (10), die Folgendes umfasst:
eine Kanüle (12) und
einen Obturator (14), der durch die Kanüle (12) aufgenommen werden kann und dafür konfiguriert ist, ein eingedrücktes Knochenfragment, das mit einer Impressionsfraktur verknüpft ist, zu manipulieren,
wobei der Obturator (14) eine stumpfe Spitze (16) einschließt, die sich über ein distales Ende (18) der Kanüle (12) hinaus erstreckt,
wobei die stumpfe Spitze (16) und das distale Ende (18) gekrümmt sind und sich entlang einer Achse erstrecken, die quer zu einer Längsachse der chirurgischen Baugruppe (10) verläuft, wobei der Obturator (14) von der Kanüle (12) entfernbar ist und wobei die chirurgische Baugruppe Folgendes einschließt:
eine Zuführungsvorrichtung (62), die zum Injizieren eines Reparaturmaterials (60) durch die Kanüle (12) konfiguriert ist, wobei die Kanüle (12) dafür eingerichtet ist, die ZuführungsVorrichtung (62) aufzunehmen, wenn der Obturator (14) entfernt ist.

2. Chirurgische Baugruppe nach Anspruch 1, wobei die stumpfe Spitze (16) abgerundet ist.

3. Chirurgische Baugruppe nach Anspruch 1, wobei die Kanüle (12) eine Röhre (15) einschließt, die sich zwischen einem Anschlussstück (22) und einem distalen Ende (18) erstreckt.

4. Chirurgische Baugruppe nach Anspruch 3, wobei die Röhre (15) einen Durchgang (20) einschließt, der sich zwischen dem Anschlussstück (22) und dem distalen Ende (18) erstreckt.

5. Chirurgische Baugruppe nach Anspruch 3, die mehrere Öffnungen (24) umfasst, die durch die Röhre (15) an dem distalen Ende (18) geformt sind.

6. Chirurgische Baugruppe nach Anspruch 1, wobei der Obturator (14) einen Schaft (25) einschließt, der sich zwischen einem Handgriff (26) und einer stumpfen Spitze (16) erstreckt.

7. Chirurgische Baugruppe nach Anspruch 6, wobei der Handgriff (26) ein Anschlussstück (28) und einen Griff (30), der sich von dem Anschlussstück erstreckt, einschließt.

8. Chirurgische Baugruppe nach Anspruch 1, wobei die Kanüle (12) ein erstes Anschlussstück (22) einschließt und der Obturator (14) ein zweites Anschlussstück (28) einschließt, das dafür konfiguriert ist, das erste Anschlussstück (22) in Eingriff zu nehmen, um den Obturator (14) mit der Kanüle (12) zu verbinden.

9. Chirurgische Baugruppe nach Anspruch 1, wobei die Zuführungsvorrichtung (62) eine Spritze und eine Röhre einschließt.

10. Chirurgische Baugruppe nach Anspruch 1, wobei das Reparaturmaterial (60) einen Knochenzement, ein Knochentransplantat, ein Knochenblock-Allotransplantat oder ein autologes Material einschließt.

11. Chirurgische Baugruppe nach Anspruch 5, wobei die mehreren Öffnungen (24) derart in Fluidverbindung mit einem Durchgang (20) der Röhre (15) stehen, dass das Reparaturmaterial (60) aus der Zuführungsvorrichtung (62) durch den Durchgang (20) und danach durch die mehreren Öffnungen (24) strömt.

## Revendications

1. Ensemble chirurgical (10), comprenant :
une canule (12) ; et
un obturateur (14) pouvant être reçu à travers ladite canule (12) et configuré pour manipuler un fragment d'os déprimé associé à une fracture de dépression ;
dans lequel ledit obturateur (14) inclut une pointe émoussée (16) s'étendant au-delà d'une extrémité distale (18) de ladite canule (12) ;
dans lequel ladite pointe émoussée (16) et ladite extrémité distale (18) sont courbées et s'étendent le long d'un axe transversal à un axe longitudinal dudit ensemble chirurgical (10) ; dans lequel ledit obturateur (14) peut être retiré de ladite canule (12), et dans lequel ledit ensemble chirurgical inclut :
un dispositif d'administration (62) configuré pour injecter un matériau de réparation (60) à travers ladite canule (12), ladite canule (12) étant adaptée pour recevoir ledit dispositif d'administration (62) quand ledit obturateur (14) est retiré.

2. Ensemble chirurgical selon la revendication 1, dans lequel ladite pointe émoussée (16) est arrondie.

3. Ensemble chirurgical selon la revendication 1, dans lequel ladite canule (12) inclut un tube (15) s'étendant entre un raccord (22) et une extrémité distale (18).

4. Ensemble chirurgical selon la revendication 3, dans lequel ledit tube (15) inclut un passage (20) s'étendant entre ledit raccord (22) et ladite extrémité distale (18).

5. Ensemble chirurgical selon la revendication 3, comprenant plusieurs ouvertures (24) formées à travers ledit tube (15) au niveau de ladite extrémité distale (18).

6. Ensemble chirurgical selon la revendication 1, dans lequel ledit obturateur (14) inclut un arbre (25) s'étendant entre une poignée (26) et une pointe émoussée (16).

7. Ensemble chirurgical selon la revendication 6, dans lequel ladite poignée (26) inclut un raccord (28) et un élément de prise (30) s'étendant à partir dudit raccord.

8. Ensemble chirurgical selon la revendication 1, dans lequel ladite canule (12) inclut un premier raccord (22), ledit obturateur (14) incluant un deuxième raccord (28) configuré pour s'engager dans le premier raccord (22) pour connecter ledit obturateur (14) à ladite canule (12).

9. Ensemble chirurgical selon la revendication 1, dans lequel ledit dispositif d'administration (62) inclut une seringue et un tube.

10. Ensemble chirurgical selon la revendication 1, dans lequel ledit matériau de réparation (60) inclut un ciment osseux, une greffe osseuse, un allogreffe à bouchon osseux ou un matériau autologue.

11. Ensemble chirurgical selon la revendication 5, dans lequel lesdites plusieurs ouvertures (24) sont en communication de fluide avec un passage (20) dudit tube (15), de sorte que ledit matériau de réparation (60) s'écoule à partir dudit dispositif d'administration (62) à travers ledit passage (20) et ensuite à travers lesdites plusieurs ouvertures (24).
